# EUROPEAN PATENT APPLICATION

(11) **EP 1 757 270 A1**
(43) Date of publication of application: **28.02.2007**
(21) Application number: 05730914.8
(22) Date of filing: 01.04.2005
(51) Int. Cl.: A61K 9/127

(54) **LIPOSOMAL FORMULATIONS**

(30) Priority: 02.04.2004 ES 200400826; 26.11.2004 ES 200402862
(71) Applicant: ITALFARMACO, S.A., E-28108 Alcobendas (ES)
(72) Inventor: CHANTRES ANTORANZ, José Ramon, E-28220 Majadahonda (ES); MOSCOSO DEL PRADO, Jaime, E-28108 Alcobendas (ES); ELORZA BARROETA, Ma. A.; c/o Fac. Farmacia, 28040 Madrid (ES); ELORZA BARROETA, B.; c/o Fac. Farmacia, 28040 Madrid (ES); CÓRDOBA DÍAZ, M.; c/o Facultad de Farmacia, 28040 Madrid (ES)
(74) Representative: de Justo Vàzquez, Jorge M.
(86) International application number: PCT/ES2005/000171
(87) International publication number: WO 2005/094790

(57) **Abstract**

The invention relates to formulations of hydrophilic substances encapsulated in liposomes, to the methods of encapsulation of the hydrophilic substances in the liposomes and the use of said formulations in the prevention and/or treatment of diseases and/or disorders in humans and animals.

## Description

This invention relates to formulations of hydrophilic substances encapsulated in liposomes, also to the method of encapsulating the hydrophilic substances in the liposomes and the use of said formulations in the prevention and/or treatment of diseases and/or disorders in humans or animals.

### BACKGROUND OF THE INVENTION

Liposomes are microscopic vesicles that have a central aqueous cavity surrounded by a lipid membrane formed by concentric bilayer(s). The liposomes can be unilamellar (that is, they have only one lipid bilayer), oligolamellar or multilamellar (that is, they have various bilayers). The multilamellar vesicles (MLVs) have a size of between 0.2µm and 10µm whilst unilamellar vesicles can be large (LUVs) or small (SUVs) with a size of between 0.02µm and 0.2µm. Their structure allows them to incorporate either hydrophilic substances (in the aqueous interior) or hydrophobic substances (in the lipid membrane).

The most important structural components of liposomes are phospholipids (PLs). It is possible to produce liposomes with specific properties through the use of phospholipids with neutral, positive or negative charge and/or varying the length and saturation level of fatty acid chains.
Similarly, the properties of the liposomes can be modified by, for example, adding cholesterol (CHOL) or other lipids to the membrane, or by modifying the number of lipid bilayers or by covalently bonding natural molecules (for example proteins, polysaccharides, glycolipids, antibodies or enzymes) or synthetic molecules (for example polyethylene glycol) to their surface.
In summary, there are several variables to be considered when designing a liposome and the results are not always predictable.

As vehicles for the administration of drugs, liposomes have, in theory, numerous advantages. As well as being composed of non-toxic components, generally non-immunogenic, non-irritant and biodegradable, they should be capable of encapsulating, retaining, transporting and releasing a large variety of therapeutic agents to target organs, thereby reducing adverse side effects.
However, on very few occasions the goal of developing optimum liposomal formulations from the technical and therapeutic point of view has been reached.

In particular, optimum liposomal vesicles should:
- Encapsulate the active ingredient in its interior at a high percentage, so that the encapsulation efficiency (ratio between quantity of drug encapsulated and lipid) is adequate from the dosage and formulation cost point of view;
- Be stable, that is retain the active ingredient encapsulated during the useful life of the formulation and, once administrated to the patient, reach the action site;
- Release the drug at the action site with a release profile adequate for the therapeutic objective, reducing systematic toxicity.

Additionally, and in the specific case of topical application, the lipids that form the liposomal vesicles must grant certain properties to the lipid bilayer so that the resulting liposomes promote the rapid penetration of the active ingredient across the corneal stratum and the localization of the desired action site (normally near to the base layer of the epidermis).

Although previous studies have been able to encapsulate hydrophilic substances (of low molecular weight and polar character) in liposomes, the retention of the active ingredient inside the liposomes remained a technical problem to be resolved as demonstrated in the following studies.
Simmons SP, Kramer PA. [J Pharm Sci 66: 984-986 (1977)*],* using multilamellar liposomes made of sphingomyelin, cholesterol and dicetyl-phosphate find that, after 40 hours at 25°C, only 20% of 5-FU initially encapsulated is still inside the liposomes.
Tsukada K, Ueda S, Okada R. [Chem. Pharm. Bull. 32: 1912-1935 (1984)*],* using liposomes made of DSPC find that, six hours after preparation, there is a 50% and 10% release of 5-FU at 37 and 25°C respectively.
Özer AY [Drug Target. Deliv. 1: 15-160 (1992)*],* studies the behaviour of OLB type liposomes in two compositions: a) soy lecithin:cholesterol hemisuccinate:cholesterol (10:1:4) and b) DPPC:cholesterol hemisuccinate:cholesterol (10:1:4). The retention capacity of the drug in question is reduced, but it depends on the formulation tested. In the type A liposome only 10% of 5-FU remains inside after 28 days at room temperature. In type B liposomes 40% of 5-FU is released in 42 days at room temperature.

Fresta M, Villari A, Publisi G, Cavallaro G [Int. J Pharm. 99:145-156 (1993)*],* study the retention capacity during 7 hours at 37°C of liposomes of different sizes and different compositions. According to the type and composition, they find that within this timeframe between 15% and 35% of initially retained 5-FU escapes.
Similarly, Elorza B, Elorza MA, Frutos G, Chantres JR. [BBA 1153: 135-142 (1993)*.],* study the release of 5-FU from inside liposomes over 60 minutes using LUV liposomes with two different compositions made of DSPC and sphingomyelin, respectively. In this time frame, more than 75% escapes from the DSPC liposomes and between 12 and 25% from the SM type.
EL Maghraby GMM, Williams AC, Barry BW. J Pharm Parmacol 53: 1069-1077 (2001), using LUV liposomes with three different compositions: SPC; SPC:Cholesterol. (1:1) and SPC:Cholate (84:16), find a rapid escape of 5-FU (more than 50% after 50 minutes), despite the fact it was suspended in a saturated solution of 5-FU. Similarly, when suspending SPC:Cholate liposomes in a solution without 5-FU, it produces an 80% release of initially encapsulated 5-FU after 5 minutes.
Finally, Mayer LD, Bally MB, Cullis PR, Ginsberg RS, Mitilenes GN, US Patent 6.083.530 (2000) propose to resolve two known problems: increase the encapsulation and ensure the antineoplastic drugs remain inside the liposomes. They use different compositions with and without cholesterol. The vesicles are LUV type and the drugs are actively charged through pH gradient. This method achieves a high encapsulation percentage and they are able to retain the active ingredient inside the liposomes. Nevertheless, on eliminating the pH gradient the release of the drug is immediate. 5-FU formulation is amongst examples that present this behaviour.
Norley S.G. et al., [J.Immunol. 136 : 681-685 (1986)], obtain a 44% retention of acyclovir after 14 days at 4°C and 48% and then 24 hours at 37°C using neutral liposomes made of egg phosphatidylcholine (PC) and cholesterol 15:1 prepared using the dehydration-rehydration vesicle (DRV) method.
Law S.L. et al., [Int.J.Pharm. 161 :253-259 (1998)], achieve a retention of acyclovir after 10 hours at 37°C of between 75% and less than 25% (depending on charge amount) for the positive charge, between 90% and 35% (depending on charge amount) for the negative charge and between 80% and 60% for the neutral. This was achieved by using MLV liposomes from egg PC and cholesterol 1.6:1, which is either neutral or charged through the adding of dicetyl phosphate or stearylamine in molar ratios between 0.15 and 1.6.
Fresta M. et al., [J.Parm.Pharmaco1.51 :565-576 (1999)] by using liposomes made of DPPC:CHOL 7:4 (rigid neutrals), DPPC:CHOL 7:4:1 (rigid negative charge) and DPPC:Cholesterol:DDAB 7:4:1 (rigid positive charge), obtain an acyclovir retention of between 50% and 30% after 8 hours at 37°C for the OLV (with minimal retention for neutrals and negatives) and between 65% and 45% after 8 hours at 37°C for the MLV (with minimal retention for neutrals and negatives).
Sarbolouki MN, Toliat T. PDA, [J Pharm Sci Technol 52: 23-27 (1998)] using liposomes made of EPC (flexible, neutral), of MLV and REV size, achieve a methotrexate retention after 30 days of 70% at 4°C; 40% at 25°C and <10% at 37°C.
Khopade AJ, et al. [Int. J. Pharm. 232: 157-162 (2002)] obtain a methotrexate retention of 35% after 8 hours at 37°C using MLV liposomes of HSPC:CHOL (neutral, rigid) and HSPC:CHOL:DCP (negative, rigid).
Norley SG, et.al., [J. Immunol 136: 681-685 (1986)] achieve a 50% retention of iododeoxyuridine after 24 hours at 4°C and a 20% retention after one hour at 37°C using liposomes made of egg PC (polyunsaturated, flexible) and cholesterol 15:1 (neutral), prepared using the DRV method.
Simmons SP, Kramer PA., [J Pharm Sci 66: 984-986 (1977)] obtain a 70% retention of floxuridine after 40 hours at 25°C using MLV liposomes made of sphingomyelin (SM):CHOL:DCP 4.8:2.8:1 (negative).
Maurer N. et al., [BBA 1374: 9-20 (1998)] using LUV liposomes made of DPPC:CHOL 55:45 (neutral, rigid), achieve a ciprofloxacin retention of 20% after 30 minutes at 25°C.
In sum, although the encapsulation of hydrophilic active ingredients has been achieved, a problem remains in ratio to their stability, that is, the retention of the active ingredient in their interior.

### SUMMARY OF THE INVENTION

Therefore, there is a need to have pharmaceutical formulations of hydrophilic active ingredients encapsulated in liposomes that have high encapsulation efficiency, are stable and have a release profile that is adequate for the therapeutic objective and is non-toxic for the patient.
The authors of this invention have found that the use of a mixture of neutral saturated phospholipids and charged saturated lipids in the preparation of liposomes allows the attainment of these objectives.
Thus they have achieved stable formulations of hydrophilic drugs, in which the active ingredient stays effectively encapsulated inside the liposomes during the necessary timescale for the preparation, storage and distribution of the corresponding pharmaceutical formulations until administration to the patient.

Similarly, and for the specific case of topical application, they have achieved that said formulations have a good diffusion in skin, reaching the epidermis and having a depot effect, without showing signs of toxicity (irritation of the skin) after repeated application on the skin. It is hoped that this also shows good diffusion in mucous.

Consequently, the use of a combination of neutral saturated PLs and charged saturated lipids allows us to produce topical compositions of hydrophilic drugs with low molecular weight encapsulated in liposomes that are highly stable, therapeutically effective (based on a good cutaneous diffusion of the drug) and safe (due to the low systemic absorption).

Additionally, the use of a mixture of neutral saturated PLs and charged saturated lipids would allow the stable and effective encapsulation in liposomes not only of drugs but also of other hydrophilic substances such as diagnostic agents, cosmetics, food substances, etc.

According to the above, the first aspect of this invention relates to liposomal formulations comprising at least one hydrophilic active ingredient encapsulated in liposomes composed of one or various lipid bilayers made of a mixture of at least one neutral saturated phospholipid and at least one charged saturated lipid.

The second aspect of this invention relates to an encapsulation procedure for hydrophilic active ingredients in liposomes, that is, the preparation method of the aforementioned liposomal formulations.

The third aspect of this invention relates to the use of said liposomal formulations in the preparation of medicines for the prevention or treatment of disorders or conditions in humans or animals.

The fourth aspect of this invention relates to the drug or veterinary formulations that contain the aforementioned liposomal formulations and at least one pharmaceutically acceptable excipient.

Prior to the description of the specific preparation, it is appropriate to define some specific terms related to the principal aspects of this invention, for clarification purposes only and are not intended to be limiting.

"Liposomal formulations" are understood to be those in which part or all of the active ingredient is encapsulated inside the liposomes.
Liposomes can be composed of one or several lipid bilayers and have different sizes.
The bilayers have a polar surface (interior and exterior) and a non-polar nucleus.

"Phospholipid" is understood to mean an amphiphile derivative of glycerol in which one of its hydroxyl groups is esterified with phosphoric acid and the other two hydroxyls are esterified with long-chain fatty acids, which may be equal or different from each other.
A saturated phospholipid will be that whose fatty acids only have simple (not multiple) carbon-carbon links.
A neutral phospholipid will generally be one in which another phosphoric acid hydroxyl is esterified by an alcohol substituted by a polar group (usually hydroxyl or amino) and whose net charge is zero.
A charged phospholipid will generally be one in which another phosphoric acid hydroxyl is esterified by an alcohol substituted by a polar group and whose net charge is positive or negative.
The meaning of the expression "charged saturated lipid", as well as including charged saturated phospholipids, also includes other amphiphile compounds whose net charge is different from zero, such as long chain hydrocarbonate derivatives, substituted by a polar group (for example amine) and derivatives of fatty acids.

In the context of this invention, hydrophilic drugs are considered to be those that show a log Pow (decimal logarithm of the octanol-water partition coefficient) of less than zero.
Similarly, low molecular weight drugs are considered to be those that have a molecular weight of less than 1000Da.

### DETAILED DESCRIPTION OF THE INVENTION

In principle, any negative saturated PL and any charged saturated lipid, natural or synthetic, can be used in the liposomal formulations of the present invention.

In a preferred embodiment, the neutral saturated PL is chosen from the group composed of derivatives of phosphatidylcholine and their combinations, for example dipalmitoylphosphatidylcholine (DPPC), distearoylphosphatidylcholine (DSPC), dimyristoylphosphatidylcholine (DMPC) and their combinations.

In another preferred embodiment, the charged saturated PL has a negative charge and is selected from a group composed of derivatives of phosphatidylglycerol, dipalmitoyl phosphatidyl glycerol (DPPG), phosphatidylserine, phosphatidylinositol, phosphatidic acid and their combinations, for example, distearoyl phosphatidyl glycerol (DSPG) and a mixture of phosphatidylserine esters with different saturated fatty acids (PS) (for example bovine cerebral PS).

In another preferred embodiment, the charged saturated lipid has a positive charge and is stearylamine (SA).

In principle, any ratio between neutral PLs and charged lipids can be used in the formulations of this invention.
In as preferred embodiment, the neutral PL/charged lipids molar ratio would be between 50/50 and 95/5, preferably between 80/20 and 95/5.

The liposomal formulations provided by this invention can also contain other lipid components such as sterols and derivatives (for example cholesterol) or sphingolipids (for example sphingomyelins and glycosphingolipids, in particular gangliosides).

Any hydrophilic substance could be encapsulated in the liposomal formulation of this invention. Preferably, said substance is a drug.
In principle, any hydrophilic substance could be encapsulated in the liposomal formulations of the present. In the case of topical application liposomal formulations, the hydrophilic drug is of low molecular weight.
In preferred embodiments, the pharmacologically active agent is selected from amongst acyclovir (ACV), iododeoxyuridine (IDUR), methotrexate, 5-fluorouracil (5-FU) and ciprofloxacin.

In a preferred embodiment ideal, the hydrophilic drug/lipids molar ratio would be between 0.01/1 and 40/1 , and more preferably it would be between 0.1/1 and 2/1.

The lower limit is determined by the low quantity of the drug that is practical for making liposomes given its intended use and can be easily determined by a person skilled in the art. The upper limit is conditioned by the crystallization concentration of the drug.

Particularly preferred formulations of liposomal 5-FU are those in which the liposomes are composed of a mixture of DSPC:DSPG and DSPC:PS phospholipids, with a neutral PL/charged PL ratio of between 85/15 and 95/5. In these formulations the 5-FU/lipids molar ratio can be between 0.2 and 1.5, preferably between 0.5 and 1.0.

Particularly preferred formulations of liposomal ACV are those in which the liposomes are formed of DSPC:DSPG and DPPC:CHOL:DPPG.

Drugs, PLs and other components of the formulations in the present invention are known to a person skilled in the art and can be obtained from commercial sources. For example, DSPG and DSPC were obtained from Chemi S.p.A., bovine cerebral PS and 5-FU from Sigma.

The liposomal formulations of the present invention can be prepared using any of the methods known to a person skilled in the art.
The size of the resulting liposomes will depend on the preparation method used. In an ideal production the liposomes are prepared according to the method set out in examples 1, 4, 6 and 11.
The resulting liposomes can be dehydrated (lyophilized) for their later formulation and/or storage and distribution.
Similarly, liposomes can be kept in suspension in aqueous solution that may or may not contain the drug. In the first case, the resulting suspension will contain part of the drug encapsulated in the interior of the liposomes and part on the exterior of the liposomes.

In the case of the liposomal 5-FU formulations of topical administration, it is preferable to use those that have part of the active ingredient in the external phase, such as those described in examples 4 and 5. In these formulations, the percentage by weight of 5-FU encapsulated in the interior of the liposomes with respect to the total amount of 5-FU would be between 20% and 100%, preferably between 35% and 55%.

The liposomal formulations provided by this invention are going to be particularly useful in the preparation of drug or veterinary compositions for the prevention and/or treatment of diseases or disorders in humans or animals.

The present liposomal formulations have shown to be particularly useful and advantageous in the prevention and/or treatment of diseases or disorders of the skin and/or mucous.

In particular, the liposomal 5-FU formulations according to this invention will be useful in the preparation of drug or veterinary compositions for the prevention and/or treatment of diseases or disorders related to cellular hyperproliferation, for example various types of cancer, pre-cancerous conditions or other afflictions in humans and animals.

The various types of cancer, include, but are not limited to, breast, bowel, gastric, pancreatic, bladder, prostate, head and neck, ovarian, cervical, endometrial, hepatic, lung, superficial base cell carcinoma, etc.
The pre-cancerous lesions include, for example, actinic keratosis, cervical dysplasia, etc.
Other disorders associated to cellular hyperproliferation include, as an example, psoriasis, human papilloma virus, etc.

The formulations of liposomal 5-FU given in this invention have shown to be particularly useful and advantageous in the topical treatment of hyperproliferative diseases or disorders of the skin and/or mucous such as actinic keratosis and psoriasis.

Similarly, the liposomal ACV formulations of the present are useful in the topical treatment of infections caused by the herpes virus in the skin and/or mucous, especially in the treatment of cutaneous herpes.

Preferably, the liposomal formulations of the present invention would be administered in an acceptable pharmaceutical vehicle that is composed of at least one excipient. Said excipient can be selected from any of those known to a person skilled in the art and according to the method of administration to be used, which will depend on the desired therapeutic objective.
For parenteral administration, the vehicle is normally composed of water and the auxiliary substances required to make it compatible with the physiological conditions such as pH regulator agents, tonicity etc. The resulting aqueous solution can be sterilised by conventional methods and bottled for use, or filtered and lyophilized, which could require the use of cryoprotector agents. The lyophilized material is combined with a sterile aqueous solution prior to administration.

For topical administration, the vehicle will be composed of excipients selected according to the drug form in question in each case, preferably liquid or semi-solid. The liquid formulations include aqueous solutions, hydro-alcoholic solutions, lotions etc. The semi-solid formulations can be ointments, creams or gels. Any of these formulations may require the use of preservatives, anti-oxidant agents, chelating agents, emulsifiers, pH regulator systems, tonicity regulating substances, conservatives, bioadhesive polymers, gelling substances, etc.
The preservative can be chosen from between, for example, sodium benzoate, ascorbic acid, parabens, bronopol, methylisothiazolinone and their combinations. The anti-oxidant can be chosen, as an example, from amongst butylated hydroxytoluene (BHT), hydroxyanisole and tocopherol and their derivatives. The chelating agent can be chosen, for example, from the group formed by ethylenediaminetetraacetic acid (EDTA), ethylene glycol bis (2-aminoethyl ether) tetraacetic acid (EGTA) and citric acid or their salts. The pH regulator system can be chosen, as an example, from between a phosphate buffer solution and a citrate buffer solution. The gelling agent can be selected, for example, from amongst Carbopols, Poloxamers, carboxymethylcellulose (CMC), hydroxyethylcellulose (HEC), Hydroxypropyl Methylcellulose (HPMC), methylcellulose (MC) and their combinations.

In a particularly preferred embodiment, the veterinary or drug compositions of this invention will be formulated for topical administration, on skin or mucous.

The dose of the pharmacologically active ingredient to be administrated to the patient depends on the therapeutic indication of the formula, on the patient's response to treatment and concurrent therapy.

### CLINICAL TRIALS

The unexpected and advantageous properties of the formulations of this invention are shown through the following non-limitative "in-vitro" and "in-vivo" trials.

### A- PREPARATION PROCEDURES AND STABILITY TRIALS

**Example 1:** Encapsulation of 5-FU in liposomes of DSPC:DSPG composition, without 5-FU in external phase.

384mg of DSPC, 43mg of DSPG, 4.5g of glass beads and 9ml of chloroform:methanol mixture at a ratio of 2:1v/v is added to a rotary evaporator flask. Once the lipids are dissolved in the solvent mixture, the flask is connected to the rotary evaporator, and the temperature of the bath is adjusted to 40°C, and the solvent evaporates at a reduced pressure (800 mbar) until a lipid film is formed on the walls of the flask. Then the pressure is reduced 100mbar and it is maintained for 1 hour. Finally, the flask is removed from the rotary evaporator and it lyophilises overnight to eliminate possible traces of organic solvent. The lipid film formed is hydrated by stirring it with 9ml of an aqueous solution of 5-FU (50mg/ml, pH 8.8) at 65°C for 1 hour. Later, the mixture is stirred for another hour at 30°C.

The liposomic suspension formed is extracted through 2 µm polycarbonate filter pores to homogenize the size of the vesicles formed. Finally, the resulting suspension is diafiltered with 6 volumes of saline phosphate buffer (pH 6; 600 mOsmol/Kg) to eliminate the 5-FU that has not been encapsulated, using Vivaflow 50 membranes with a 100KD cut off (Sartorius). The resulting liposomes are diluted with the same buffer, is separated into polypropylene vials and they are stored at a 25°C until analysis.

To determine the total amount of 5-FU present in the liposomic suspension the suspension is previously diluted in methanol to dissolve the lipids of the liposomic membrane and release the content. The concentration of 5-FU is determined later through a spectroscopy at 266nm and compared with a calibration curve.

To determine the encapsulated fluorouracil, the liposomes are previously sedimented through centrifuge for 25 minutes at 26,000g, collecting the supernatant. Later the 5-FU content is analysed in the supernatant using the procedure set out above.

The difference between the total 5-FU and that which is present in the supernatant represents the fraction encapsulated. In the example described, the concentrate of total 5-FU came to 0.800mg/ml, which was 95.5% of that which was encapsulated inside the liposomes at the beginning of the study. The 5-FU/lipid molar ratio was 0.82.

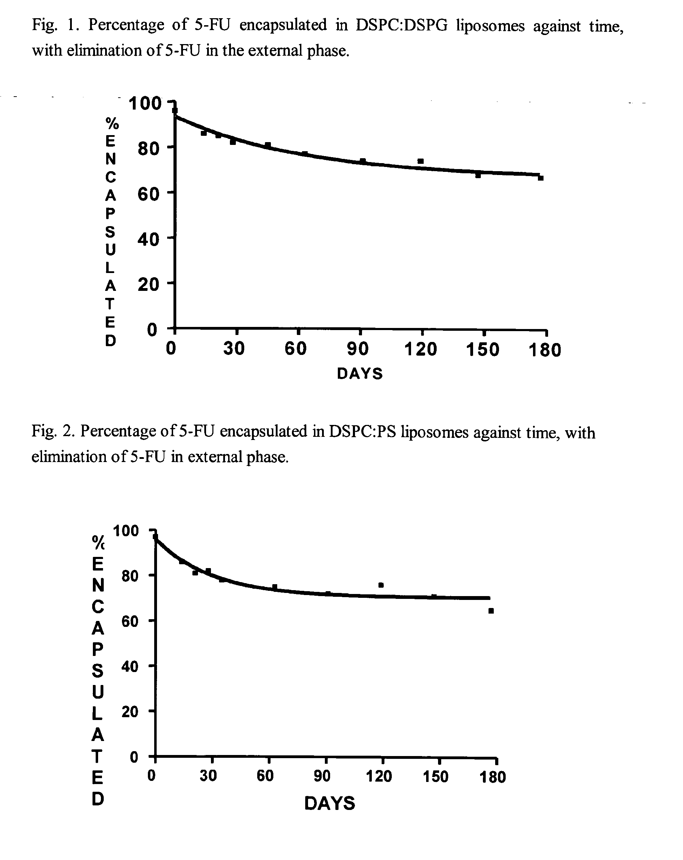

Figure 1 shows the evolution in the percentage of encapsulated 5-FU (expressed in percentage of the initial) over 6 months at 25°C. An initial decrease in the encapsulated 5-FU is shown, which then stabilises at 67% (C.I. 95% = 61 - 73). Thus it demonstrates that using a combination of neutral saturated and charged saturated PLs stable liposomal 5-FU formulations are achieved.

**Example 2:** Encapsulation of 5-FU in DSPC:PS liposomes, without 5-FU in the external phase.

This is carried out in a similar way to the example but by substituting DSPG with PS as negatively charged phospholipid. In this case, the concentration of total 5-FU came to 0.600mg/ml, with 96.5% initially encapsulated and a 5-FU/lipid molar ratio of 0.7.

Figure 2 shows the evolution in the percentage of encapsulated (expressed in percentage of the initial) over 6 months at 25°C. The kinetics are similar to those observed in the previous example, which stabilises at 71% of encapsulated 5-FU (C.I. 95% = 66 - 75).

**Example 3:** Encapsulation of 5-FU in DSPC:DSPG liposomes, with partial elimination of 5-FU in the external phase.

This is carried out in a similar way to example 1 but carrying out the diafiltration with only 3 volumes of buffer, so that not all of the non-encapsulated 5-FU is eliminated. The suspension of liposomes is diluted until a concentration of total 5-FU of 3.41mg/ml is reached, 71% of which is encapsulated inside the liposomes, with a 5-FU/lipid molar ratio of 1.5.

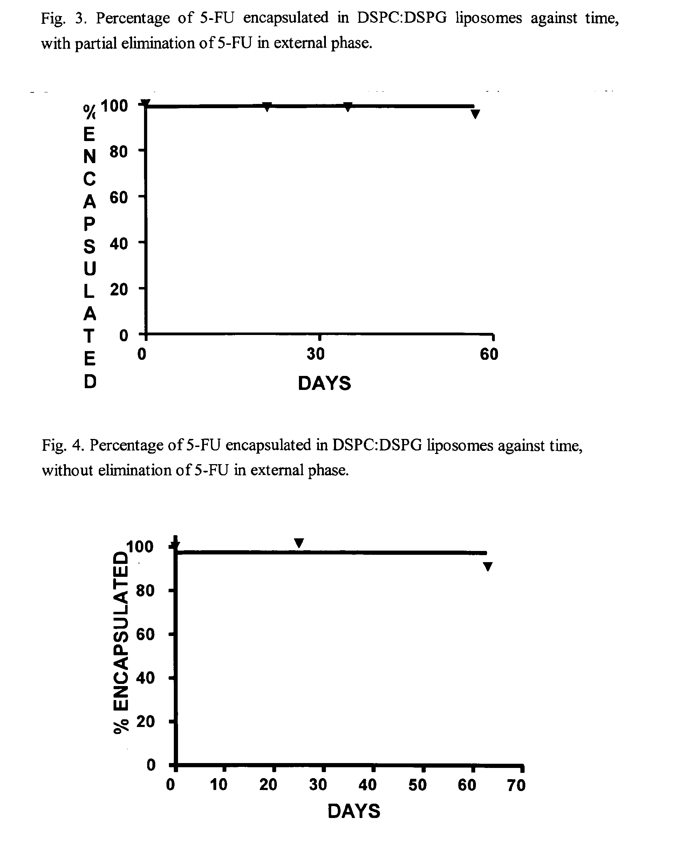

A follow up of the stability is carried out over two months at 25°C. The results are shown in figure 3. In these conditions no significant decrease in the percentage encapsulated in the timeframe studied.

**Example 4:** Encapsulation of 5-FU in DSPC:DSPG liposomes, without partial elimination of 5-FU in the external phase.

14.0 g of DSPC, 1.55mg of DSPG, 30g of glass beads and 60ml of chloroform:methanol mixture at a ratio of 2:1v/v is added to a rotary evaporator flask. Once the lipids are dissolved in the solvent mixture, the flask is connected to the rotary evaporator, and the temperature of the bath is adjusted to 40°C, and the solvent evaporates at a reduced pressure (800mbar) until a lipid film is formed on the walls of the flask. Later the pressure is reduced 100mbar and it is maintained for 1 hour. Finally, the flask is removed from the rotary evaporator and it lyophilizes overnight to eliminate possible traces of organic solvent. The lipid film formed is hydrated by stirring it with 60ml of solution of 30mg/ml of 5-FU in 10mM citrate buffer, pH6 preheated to 65°C for 1 hour. Later, and maintaining the temperature at 65°C, the resulting suspension is homogenized using an Ultraturrax T-25 (IKA), for 15 minutes at 6500rpm. Later, the liposomic suspension is diluted with a saline citrate buffer (300 mOsmol/Kg) until the concentration nears 12 mg/ml de 5-FU and it is stirred for another hour at room temperature. The resulting suspension is aliquoted and stored at 25°C until analysis.

The quantification of 5-FU is carried out in a similar way to the previous examples. The measuring of the percentage of encapsulation is carried out by first eliminating the 5-FU that is not encapsulated by exclusion chromatography in Sephadex G-50 columns (PD-10, Amersham) and measuring the amount of 5-FU in the liposomic fraction, which corresponds to the exclusion amount of the column. In this case the liposomic preparation has a total 5-FU concentration total of 11.6mg/ml, with 36% initially encapsulated, with a 5-FU/lipid molar ratio of 0.7.

Figure 4 shows the results of the follow up of the encapsulation during 70 days at 25°C, in which no significant decrease was observed.

**Example 5:** Encapsulation of 5-FU in DSPC:DSPG liposomes, without partial elimination of 5-FU in the external phase.

This is carried out in a similar way to Example 4, substituting the citrate buffer for a 10mM saline phosphate buffer, pH 6.2. The suspension produced has 12.5mg/ml of total 5-FU, 41% of which is encapsulated in liposomes, with a 5-FU/lipid molar ratio of 0.68.

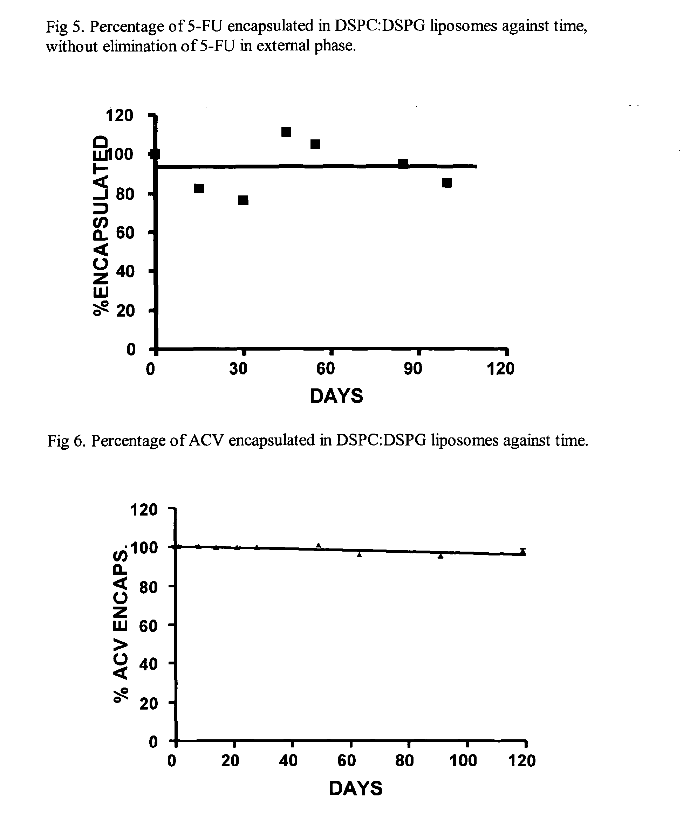

Figure 5 shows the results of the follow up of the encapsulation over 4 months at 25°C, no significant decline was observed.

**Example 6:** Encapsulation of ACV in MLV DSPC:DSPG liposomes.

A 9:1 molar ratio of DSPC and DSPG and a chloroform:methanol mixture at a ratio of 2:1 v/v is added to a rotary evaporator flask. Once the lipids are dissolved in the solvent mixture, the flask is connected to the rotary evaporator, and the temperature of the bath is adjusted to 40°C, and the solvent evaporates at a reduced pressure (800mbar) until a lipid film is formed on the walls of the flask. Later the pressure is reduced 100mbar and it is maintained for 1 hour. Finally, the flask is removed from the rotary evaporator and it lyophilizes overnight to eliminate possible traces of organic solvent. The lipid film formed is hydrated by stirring it with 9ml of a sodium ACV aqueous solution (50 mg/ml) at 65°C for one hour in the rotary evaporator.

The multilamellar liposomes formed are processed through extrusion using 2.0 µm polycarbonate filter pores. Then, the non-encapsulated ACV is eliminated with diafiltration, first with three volumes of a 150mM NaCl solution, pH 11.5 and then using 5-6 volumes of 10mM phosphate buffer pH 6.0, NaCl 150mM, using Vivaflow 50 membranes with a 100KD cut off (Sartorius). The resulting liposomes are diluted with the same buffer, the solution is fractioned in aliquots which are stored in polypropylene vials at 25°C until analysis.

To determine the total amount of ACV present in the liposomic suspension the suspension is firstly diluted in methanol to dissolve the lipids of the liposomic membrane and release the content. Then, the concentration of ACV is determined later through a spectroscopy at 254nm and compared with a calibration curve.

To determine the encapsulated ACV, the liposomes are previously sedimented through centrifuge for 25 minutes at 26,000g, collecting the supernatant. Later the ACV content is analysed in the supernatant using the procedure set out above.

The difference between the total ACV and that which is present in the supernatant represents the fraction encapsulated. The concentration of total ACV came to 0.555mg/ml, which was 96.3% of that which was encapsulated inside the liposomes at the beginning of the study. The ACV/lipid molar ratio was 0.83.

Figure 6 shows the evolution in the percentage of encapsulated ACV (expressed in percentage of the initial) during 4 months at 25°C, no significant decrease was detected. Thus demonstrating that using a combination of neutral saturated and charged saturated PLs stable Liposomal ACV formulations are achieved.

**Example 7:** Encapsulation of ACV in DPPC:CHOL:DPPG LUV liposomes.

A 60:10:40 molar ratio of DPPC, DPPG and CHOL and a chloroform:methanol mixture at a ratio of 2:1v/v is added to a rotary evaporator flask and the procedure used for example 6 is repeated. The lipid film formed is hydrated by stirring it with 9ml of a sodium ACV aqueous solution (50 mg/ml) at 55°C for one hour in the rotary evaporator

The liposomes produced are processed in a similar way to those in example 6, except that the extrusion is through 0.2µm pore membranes.

The determination of total and encapsulated ACV is carried out using the same procedure as in example 6. The resulting liposomes showed an ACV/lipid molar ratio of 0.52, with 98.4% of ACV encapsulated inside the lipsomes. No variation in the percentage of encapsulated ACV over 6 weeks at 25°C was detected.

**Example 8:** Encapsulation of ACV in LUV liposomes made of Palmitoyl oleoyl phosphatidylcholine (POPC).

The liposomes are prepared as in example 7, substituting the lipids for POPC.

The determination of encapsulated ACV is carried out following the method set out in example 7, checking that by using an unsaturated neutral PL, only 27.3% of the initial ACV is retained inside the liposomes after 24 hours, that is that the liposomal formulation is not stable.

**Example 9:** Encapsulation of ACV in LUV POPC:DPPG liposomes.

The liposomes are prepared as in example 7, substituting the lipids for POPC and DPPG in molar ratio 9:1.

The determination of encapsulated ACV is carried out following the method set out in example 7, checking that by using an unsaturated neutral PL, only 38.1% of the initial ACV is retained inside the liposomes after 24 hours.

**Example 10:** Unstable encapsulation of ACV in LUV DPPC:CHOL liposomes.

The liposomes are prepared as in example 7, substituting the lipids for DPPC and cholesterol at a molar ratio of 6:4. The determination of encapsulated ACV is carried out following the method set out in example 7, checking that by using a mixture of lipids that do not have a charged saturated PL only 25.4% of initial ACV is retained inside the lipsomes after 24 hours.

**Example 11:** Encapsulation of IDUR in MLV DSPC:DSPG liposomes.

A 9:1 molar ratio of DSPC and DSPG and a chloroform:methanol mixture at a ratio of 2:1v/v is added to a rotary evaporator flask. Once the lipids are dissolved in the solvent mixture, the flask is connected to the rotary evaporator, and the temperature of the bath is adjusted to 40°C, and the solvent evaporates at a reduced pressure (800mbar) until a lipid film is formed on the walls of the flask. Later the pressure is reduced 100mbar and it is maintained for 1 hour. Finally, the flask is removed from the rotary evaporator and it lyophilizes overnight to eliminate possible traces of organic solvent. The lipid film formed is hydrated by stirring it with 50ml iododeoxyuridine aqueous solution (2 mg/ml) (50 mg/ml) at 65°C for one hour in the rotary evaporator

The liposomic suspension formed is extracted using 2µm polycarbonate filter pores to homogenize the size of the vesicles formed. Then, the non-encapsulated drug is eliminated with diafiltration, using 6 volumes of saline phosphate buffer (pH 6; 150 mM NaCl) using Vivaflow 50 membranes with a 100KD cut off (Sartorius). The resulting liposomes are diluted with the same buffer, are separated into polypropylene vials and they are stored at a 25°C until analysis.

To determine the total amount of IDUR present in the liposomic suspension the suspension is firstly diluted in methanol to dissolve the lipids of the liposomic membrane and release the content. Then, the concentration of IDUR is determined later through a spectroscopy at 284nm and compared with a calibration curve.

To determine the encapsulated IDUR, the liposomes are previously sedimented through centrifuge for 25 minutes at 26,000g, collecting the supernatant. Later the IDUR content is analysed in the supernatant using the procedure set out above.

The difference between the total IDUR and that which is present in the supernatant represents the fraction encapsulated. In the example described the concentration of total IDUR came to 0.555mg/ml, which was 97.8% of that which was encapsulated inside the liposomes at the beginning of the study. The IDUR/lipid molar ratio was 0.04.

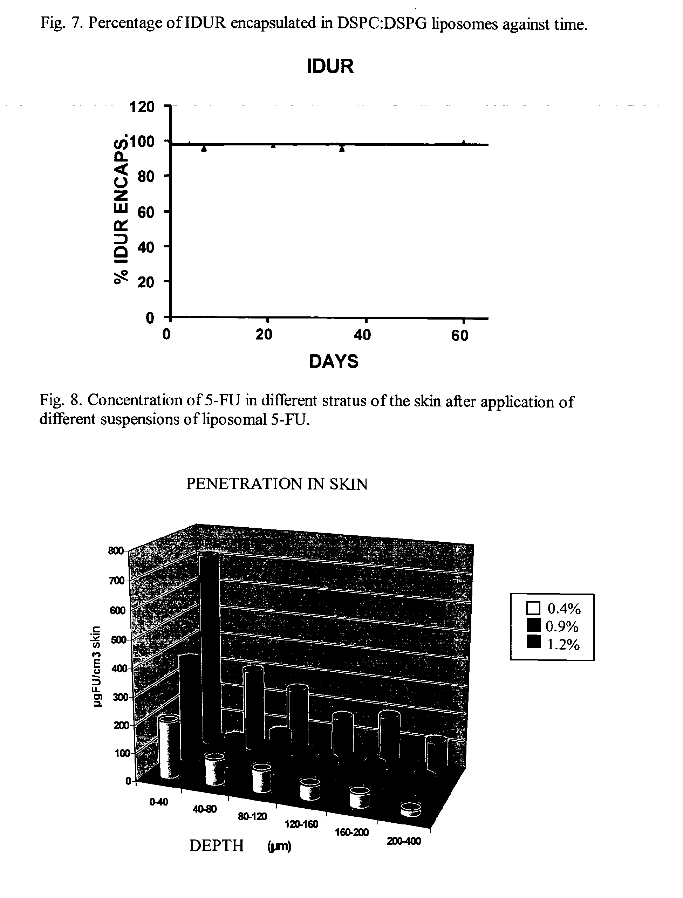

Figure 7 shows the evolution in the percentage of encapsulated IDUR (expressed in percentage of the initial) over 2 months at 25°C, no significant decrease was detected. Thus demonstrating that using a combination of neutral saturated and charged saturated PLs stable IDUR liposomal formulations are achieved.

### B- DIFFUSION IN SKIN

Example 12: Cutaneous penetration and dosage ratio response to liposomal 5-FU.
The liposomes are prepared according to example 5 with which three different dilutions are obtained using a saline phosphate buffer pH 6.2 until reaching a total content of 5-FU of 1.2%, 0.9% and 0.4%, respectively. The percentage of encapsulated 5-FU is 41 %.

With each one of the three concentrations diffusion experiments are carried out as described below, during 24 hours. Each liposome sample is analysed in 8 different experiments.

The diffusion studies are carried out using skin from pigs' ears dermatomized to 1mm, mounted on static Franz diffusion cells (Hanson Research), with a total diffusion surface of 0.636cm². The receptor of each one of the diffusion is filled with 4.5ml of phosphate buffer and is kept at 37°C with a rotation of 300 rpm. 10µl of the liposomic suspension is added to the surface of the skin. After the established time for diffusion, the cells are dismounted, the surface of the skin is cleaned extensively to eliminated excess liposomic suspension. After washing, the skin is dried out and frozen embedded in Tissue-Tek (Bayer) and are placed in criostat (CM-1900, Leica). Parallel 40µm sequential cuts are obtained on the surface of the skin. The 5-FU present in the cuts in the skin is extracted during 15 minutes with 300µl of water at 50°C. Later the samples are centrifuged (10 minutes 8000rpm; Centrifuge 5804R, Eppendorf) and are filtered (0.45µm) for HPLC analysis.

The analysis of the 5-FU content in the samples of the receptor compartment and the cuts in the skin are carried out using HPLC (chromatograph HP1100 with visible-UV detector HP1100; Hewlett-Packard), using the following chromatographic conditions:
- Kromasil column C₁₈ (240x4) 5µm
- ODS pre-column
- Pre-column filter
- Mobile phase: K₂HPO₄ 0.01 M (pH = 3) : Methanol (98:2)
- Flow = 1ml/min
- Temperature = 35°C
- Detection = 266nm
- Injection volume = 100µl

Figure 8 shows the concentration of 5-FU in the different stratus of the skin.
The first 80µm correspond to the corneal stratus, the epidermis is found between 80 and 200 and below, the dermis (Jenning, V., Gysler, A., Schäfer Korting, M., Gohla, S.H. Eur. J. Pharm. Biopharm., 49. 211-218 (2000)).

There is a clear penetration of 5-FU in all the stratus of the skin analysed, with an evident dosage-response effect.

**Example 13:** Cutaneous penetration of liposomal ACV and comparison with ACV in solution.

The liposomes are prepared according to example 7 and the resulting suspension is adjusted until reaching a ACV concentration of 1mg/ml. Simultaneously, a reference solution of 1mg/ml ACV is prepared in the same buffer as the liposomes. Both the liposome suspension and the ACV reference suspension are studied in diffusion experiments as described below

The diffusion experiments are carried out in continuous flow equipment with PermeGear ILC-07 cells with a 0.69cm² surface, using dorsal skin of fur-less mice (SKH1). In order to standardize the data, a fixed concentration of ACV of 1mg/ml is used, dilutions of the liposomal suspensions prepared according to example 2 are used. A solution of ACV in the same buffer is used as a reference. All the experiments are carried out placing 200µl of the liposomal solution or suspension de in the donating cell.

The mouse skin samples, after eliminating the subcutaneous oil, are placed on diffusion cells. A 10mM phosphate buffer, 150mM NaCl, pH 6 is used as a receptor liquid (in contact with the dermis). The buffer, kept at 37°C, is circulated in the diffusion chamber at a constant flow of 0.5ml/h. All the samples are hydrated for a minimum of one hour before starting the diffusion, which takes place for a total of 18 hours. After this time, the skin membranes are washed three times with isotonic phosphate buffer pH 6, and are carefully dried with a cotton tip. The ACV present in the whole skin and in the epidermis is analysed. For the latter, once the diffusion has taken place, the membranes of skin are washed and dried as described and the corneal stratus is eliminated by applying and removing ten times consecutively adhesive tape on the surface of the skin.
Once washed and cut, the skin or epidermis are frozen and defrosted repeatedly in liquid nitrogen, and 1ml de 0.5 N NaOH is added. The suspension obtained is incubated for 16 hours at 50°C for its solubilization. Finally, the samples are centrifuged and the solution is removed avoiding the surface oily layer and they are filtered through nylon 0.2µm filters. At this point the samples are frozen until processing prior to analysis.

Said processing is carried out by diluting the samples in 0.5 N NaOH and precipitating the proteins with 5N HClO4. Later they are centrifuged and the supernatant is collected by filtering it with 0.2µm nylon filters directly into HPLC injection vials. The analysis of the samples is carried out through HPLC, using a Discovery Amide C16 column of 250 x 4.6 mm and 5µm particles (Supelco) with the following chromatographic conditions:
■ Eluent: Water
■ Flow: 1.5 ml/min
■ Detection: 254 nm
■ Temperature: 27°C
■ Injection volume: 50µl

The results are shown in table 1, where you can see that the liposomes according to the invention have an important promoter effect on the diffusion of ACV in skin, the encapsulated ACV reaching the deeper layers of the viable skin further than the corneal stratus in quantities of between 3 and 4 times greater than those produced with the drug in solution.

**Table 1. Levels of ACV in skin after 18 hours of diffusion (% applied dose + S.D.)**

| | **N (each group)** | **Liposomal ACV** | **ACV in solution** | **Differential** |
|---|---|---|---|---|
| **Total Skin** | 12 | 0.89 ± 0.40 | 0.21 ± 0.18 | P = 0.0131 |
| **Epidermis** | 6 | 0.37 ± 0.19 | 0.13 ± 0.7 | P < 0.0001 |

**Example 14:** Comparison with a commercial formulation of sustained release of 5-FU.

Liposomes are obtained according to example 5 and a dilution with a 0.4% total content of 5-FU is prepared.

Diffusion experiments are carried out according to the method described in example 12 with this preparation and with a commercial preparation of the Carac® brand, that contains 0.5% of 5-FU, 2/3 parts of which are contained in microsponges. It is stated that this formulation has a sustained release of 5-FU. The experiments are carried out over 2, 6, 24 and 72 hours. In all cases the amount of product applied was 10mg.

The average levels measured in skin are always higher with the liposomic formulation, and those at 24 hours are statistically significant in epidermis and dermis. A clear depot effect is observed with the liposomes, with the concentration of 5-FU in epidermis remaining constant between 2 and 24 hours.

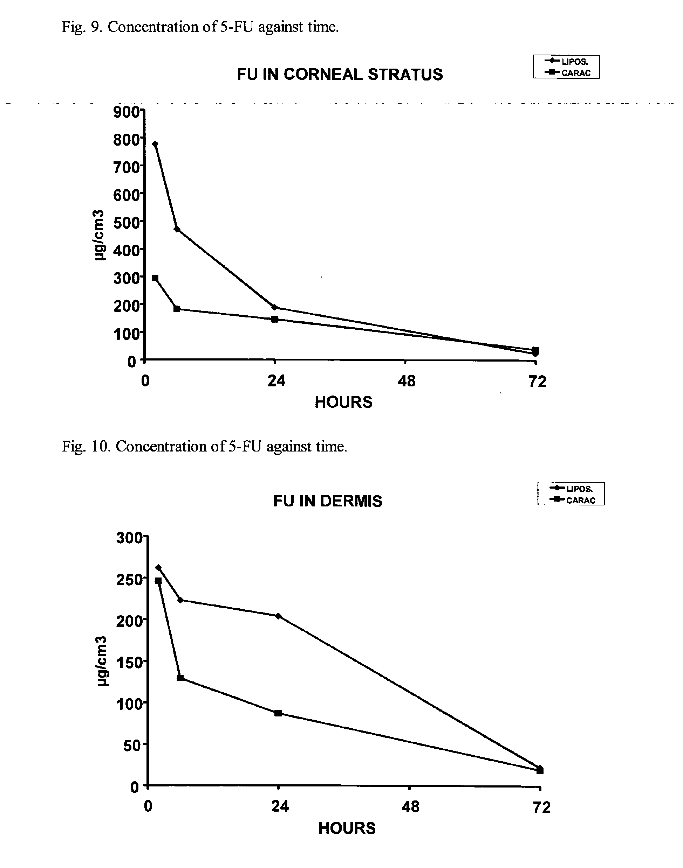

The results are shown in figures 9, 10 and 11 referring to the corneal stratus, epidermis and dermis respectively.

**Example 15:** Dermatological irritation of liposomal 5-FU and comparison with a commercial formulation of 5-FU.

A 1.2% 5-FU liposomic suspension is used as described in example 6. Dermatological irritation experiments were carried out with this composition and with a formulation commercially available of the Efudix® brand, with 5% 5-FU content.

The dermatological irritation experiment is carried out following a process based on guide 404 of the OCDE. Three male albino (New Zealand) rabbits are used (KBL IOPS/SPF; Charles River Ibérica, S.A.) with a weight of between 1.8-2 kg.

The animals are kept in individual cages with free access to a standard diet and water. The animals are kept in controlled conditions in relation to temperature (20 ±2°C), photoperiods (12/12 hours light/dark), and relative humidity (40-60%).

A quantity of 0.5ml of the liposomic suspension of 5-FU at 1.2% described in example 6 are applied topically on an area of approximately 6 cm² of previously shaved skin which are then covered in gauze. After 4 hours of application the patch is removed and the application area is washed. After one hour and 24 hours the area is observed and the irritation produced is observed and the grade of erythema and edema respectively is noted on a scale of 0 to 4. This application is repeated in the same zone of intact skin daily, five days per week, for a total of 3 weeks. The same procedure is carried out with the commercial formulation of 5% 5-FU.

After each application, the average markings for each sign of irritation are obtained in the observations carried out after one hour and after 24 hours, noting separately from 0 to 4 the erythema and edema produced, and classifying the irritation grade according to the EU dermatological classification, in which the substances that produce markings of>2 in edema or in erythema are considered irritants.

In these conditions Efudix® has shown to be highly irritant, obliging the interruption of the treatment after 2 weeks due to the severity of the irritation produced. Conversely, the liposomic suspension has shown to be non-irritant for the entire duration of the treatment.

### GALENIC FORMULATIONS

The liposomes of this invention can be transported to various pharmaceutical compositions, some of which are listed below, although it is not an exhaustive list.

| **A.- Lotion** | |
|---|---|
| Liposomal suspension | q.s. reach desired dose of drug |
| Glycerine | 50 g |
| Disodium phosphate | 0.044 g |
| Monosodium phosphate | 0.124 g |
| Kathon® CG | 0.05 g |
| Water | q.s. complete formulation |

| **B.- Gel** | |
|---|---|
| Liposomal suspension | q.s. reach desired dose of drug |
| Carbopol® 980 ¹ | 0.5 - 1g |
| Triethanolamine | q.s. pH = 6.5 |
| Methylisothiazolinone (Kathon®CG) | 0.05 g |
| Propylene glycol | 0.5 g |
| Glycerine | 15 g |
| Water | q.s. complete formulation |

Instead of Carbopol, another gelling agent can be chosen from amongst Poloxamer 407 in quantities of between 15 and 20%, carboxymethylcellulose (CMC) between 1 and 10%, hydroxyethylcellulose (HEC) between 1 and 5%, hydroxypropylmethylcellulose (HPMC) between 1 and 15%, methylcellulose (MC) between 1 and 5% in weight of the formulation and their combinations.

| **Liposomal 5-FU Gel** | |
|---|---|
| 5- FU | 0.4% |
| DSPC | 1.2% |
| DSPG | 0.13% |
| Na₂HPO₄.2H₂O | 0.035% |
| NaH₂PO₄ | 0.1075% |
| EDTANa₂ | 0.1 % |
| NaCl | 0.76% |
| Propylene glycol | 2% |
| EtOH 96° | 2% |
| HEC | 2% |
| Bronopol | 0.04% |
| Water | q.s. 100% |

Propylene glycol (PG) may be in quantities of between 1 and 20%, ethanol between 1% and 5%, hydroxyethylcellulose (HEC) between 1% and 5%, bronopol between 0.01 and 0.1 % in weight of the formulation.

Instead of the phosphate buffer Na2HPO4.2H2O / NaH2PO4 other pharmaceutically acceptable pH regulating systems can be used, known to a person skilled in the art, in normal concentrations. If necessary, the buffer and NaCl quantities can be modified in a way known to a person skilled in the art, in order to make the formulation isotonic.

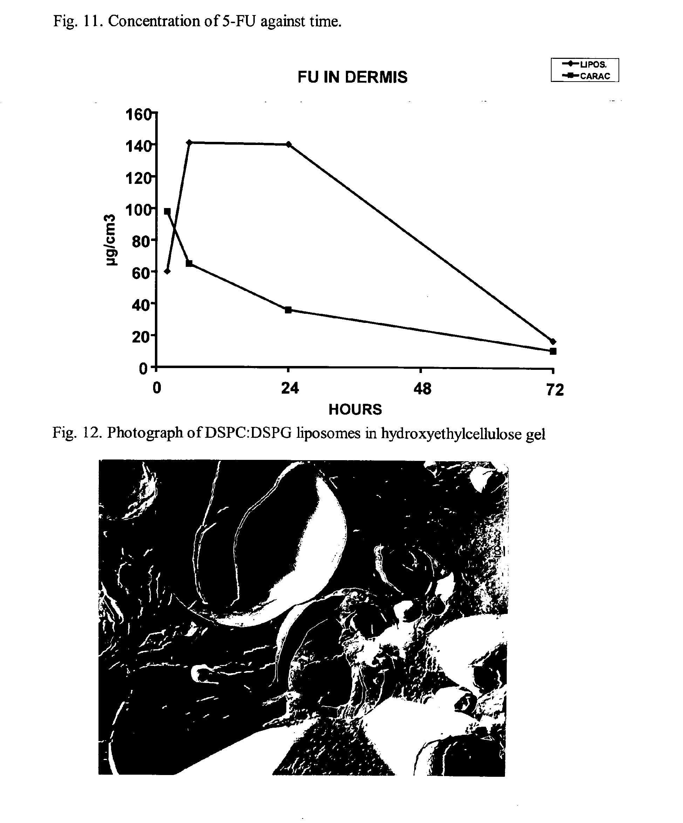

The structure of the lipsomes according to this invention are maintained in gels of this type, as shown in figure 12 where the photograph of DSPC:DSPG liposomes in 2% hydroxyethylcellulose gel is shown obtained by freeze-fracture electron microscopy.

### C.- Parenteral Solution

Liposome suspension
Saline phosphate buffer
Sodium chloride isotonic solution

## Claims

1. Liposomal formulations comprising at least one active hydrophilic agent encapsulated in liposomes composed of at least one lipid bilayer formed by a mixture of at least one neutral saturated phospholipid and at least one charged saturated lipid.

2. Liposomal formulations according to claim 1, **characterised in that** the neutral saturated phospholipid is chosen from amongst derivatives of phosphatidylcholine and their combinations.

3. Liposomal formulations according to claim 2, **characterised in that** the derivative of phosphatidylcholine is chosen from amongst DSPC, DPPC and DMPC.

4. Liposomal formulations according to claim 1, **characterised in that** the negatively charged saturated lipid is chosen from amongst a group composed of derivatives of phosphatidylglycerol, phosphatidylserine, phosphatidylinositol, phosphatidic acid and their combinations.

5. Liposomal formulations according to claim 4, **characterised in that** the negatively charged saturated lipid is chosen from amongst DSPG, DPPG and PS.

6. Liposomal formulations according to claim 1, **characterised in that** the positively charged saturated lipid is SA.

7. Liposomal formulations according to claims 1 to 6, that can also contain at least one other lipid chosen from amongst sterols and derivatives, gangliosides and sphingomyelins.

8. Liposomal formulations according to claim 7, **characterised in that** the sterol is cholesterol.

9. Liposomal formulations according to claim 1 **characterised in that** the active hydrophilic agent is a drug.

10. Liposomal formulations according to claim 9, **characterised in that** the drug has low molecular weight.

11. Liposomal formulations according to claim 10, **characterised in that** the drug with low molecular weight is selected from amongst 5-fluorouracil, acyclovir, iododeoxyuridine, methotrexate and ciprofloxacin.

12. Liposomal formulations according to the previous claims, comprising 5-FU encapsulated in liposomes composed of DSPC:DSPG.

13. Liposomal formulations according to claims 1 to 11, comprising 5-FU encapsulated in liposomes composed of DSPC:PS.

14. Liposomal formulations according to claims 1 to 11, comprising ACV encapsulated in liposomes composed of DPPC:CHOL:DPPG.

15. Liposomal formulations according to claims 1 to 11, comprising ACV encapsulated in liposomes composed of DSPC:DSPG.

16. Liposomal formulations according to the previous claims, **characterised in that** the bilayer lipids have a neutral saturated PLs/charged saturated lipid molar ratio between 50/50 and 95/5.

17. Liposomal formulations according to claim 16, **characterised in that** the neutral saturated PLs/charged saturated lipid molar ratio is between 80/20 and 95/5.

18. Liposomal formulations according to the previous claims, **characterised in that** the active hydrophilic agent/lipids molar ratio is between 0.01/1 and 40/1.

19. Liposomal formulations according to claim 18, **characterised in that** active hydrophilic agent/ lipids molar ratio is between 0.1/1 and 2/1.

20. Liposomal formulations according to claims 12, 13, 18 and 19, **characterised in that** the 5-FU / lipid molar ratio is between 0.2 and 1.5.

21. Liposomal formulations according to claim 20, **characterised in that** the 5-FU/lipid molar ratio is between 0.5 and 1.0.

22. Pharmaceutical formulations that contain liposomal formulations according to any of claims 1 to 21 and a pharmaceutically acceptable vehicle.

23. Pharmaceutical formulations according to claim 22, for the topical administration of pharmaceutically active ingredients.

24. Use of the formulations according to any of claims 1 to 21, in the preparation of a drug for the prevention and/or treatment of diseases or disorders in humans or animals.

25. Use according to claim 24 in which the disease or disorder affects the skin and/or mucous.

26. Use according to claim 25 of a liposomal formulation of 5-FU according to claims 12, 13, 16, 17, 20 or 21 in the preparation of a topical drug for the prevention and/or treatment of hyperproliferative diseases or disorders in the skin and/or mucous.

27. Use according to claim 25 of a liposomal formulation of ACV according to any of claims 14 to 19 in the preparation of a topical drug for the prevention and/or treatment of infections caused by the herpes virus in the skin and/or mucous.

28. Preparation procedures of liposomal formulations according to claims 1 to 21 that includes:
- Dissolving the lipids in a mixture of organic solvents;
- Eliminating the solvents until forming a lipid film in the walls of the container;
- Hydrating the film by stirring it with an aqueous solution of the active ingredient;
- If desired, extract the liposomic suspension formed through filters to select the vesicular size;
- Subjecting the resulting suspension to diafiltration with a buffer solution;
- If desired, dilute the liposomic suspension with a buffer solution.
